(19) Europäisches Patentamt
European Patent Office
Office européen des brevets

(11) **EP 4 170 666 A1**

(12) **EUROPEAN PATENT APPLICATION**

(43) Date of publication:
26.04.2023 Bulletin 2023/17

(21) Application number: **21204279.0**

(22) Date of filing: **22.10.2021**

(51) International Patent Classification (IPC):
**G16H 20/30** (2018.01)    **G16H 50/20** (2018.01)
**G16H 50/30** (2018.01)

(52) Cooperative Patent Classification (CPC):
**G16H 20/30; G16H 50/20; G16H 50/30**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SARTOR, Francesco**
**Eindhoven (NL)**

• **VAN BOCHOVE, Jean Luc**
**Eindhoven (NL)**
• **FONSECA, Pedro Miguel Ferreira dos Santos da**
**Eindhoven (NL)**
• **DENISSEN, Adrianus Johannes Maria**
**Eindhoven (NL)**

(74) Representative: **Philips Intellectual Property &
Standards**
**High Tech Campus 52**
**5656 AG Eindhoven (NL)**

(54) **DEVICE, SYSTEM AND METHOD FOR GENERATING INFORMATION ON RECOVERY OF A SUBJECT**

(57)    The present invention relates to a device, system and method for generating information on recovery of a subject. The information on recovery is generated by determining from the obtained one or more physiological signals and/or the obtained actual exercise data if the subject is in an exercise state in which the subject performs an exercise or in a sleep state in which the subject is sleeping, determining, from the one or more physiological signals and/or the one or more actual exercise data measured during a sleep state and the obtained subject-related information, an actual sleep architecture of the subject, estimating, from the one or more physiological signals and/or the one or more actual exercise data measured during an exercise state and the obtained subject-related information, an estimated sleep architecture of the subject, and determining first recovery information on the subject's recovery based on a comparison of the actual sleep architecture and the estimated sleep architecture.

FIG.3

EP 4 170 666 A1

**Description**

FIELD OF THE INVENTION

The present invention relates to a device, system and method for generating information on recovery of a subject.

BACKGROUND OF THE INVENTION

Training and recovery are known to be key elements to improve performance. Assessing physical recovery from exercise training is a well-known need in sport industry since optimal recovery leads to better performance Exercise episodes (including training and races) are modelled by the use of intensity indicators (e.g. heart rate zones) and duration (e.g. clock). Moreover, the pattern of such intensity and duration bouts determine the type of training (e.g. continuous long duration low intensity, high intensity interval training, etc.). These three aspects (intensity, duration, type) are used to plan training programs and to estimate/model recovery.

Recovery is as important as training for the simple reason that insufficient recovery would lead to non-functional overreaching which can lead to the overtraining syndrome. The overtraining syndrome is the condition when the physiology of the user is chronically compromised such that training sessions does not correspond to increases or maintenances of performance but rather induce a decline in performance. The occurring of overtraining syndrome is not obvious. Thus, it is important to monitor unbalances between training and recovery.

SUMMARY OF THE INVENTION

It is an object of the present invention to improve the assessment of recovery and enable the monitoring and further optimizing of the balance between training and recovery.

In a first aspect of the present invention device, system and method for generating information on recovery of a subject is presented comprising:

a sensor input configured to obtain one or more physiological signals and/or actual exercise data of a subject;
a subject data input configured to obtain subject-related information on one or more characteristics of the subject;
a processor configured to generate information on recovery of the subject using the obtained one or more physiological signals and the obtained subject data; and
an output configured to output the generated information on recovery,

wherein the processor is configured to

- determine from the obtained one or more physiological signals and/or the obtained actual exercise data if the subject is in an exercise state in which the subject performs an exercise or in a sleep state in which the subject is sleeping,
- determine, from the one or more physiological signals and/or the one or more actual exercise data measured during a sleep state and the obtained subject-related information, an actual sleep architecture of the subject,
- estimate, from the one or more physiological signals and/or the one or more actual exercise data measured during an exercise state and the obtained subject-related information, an estimated sleep architecture of the subject, and
- determine first recovery information on the subject's recovery based on a comparison of the actual sleep architecture and the estimated sleep architecture.

In a further aspect of the present invention system for generating information on recovery of a subject is presented comprising:

a sensor configured to measure one or more physiological signals and/or actual exercise data of a subject;
a subject data source configured to input and/or store subject data including information on one or more characteristics of the subject;
a device as disclosed herein configured to generate information on recovery of a subject using the measured one or more physiological signals and the subject data; and
a user interface configured to issue the generated information on recovery.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

Conventionally, the assessment of recovery on exercise training workload determination. This has the limitation to consider mainly the fatiguing effect of exercise training and how long fatigue takes to be recovered. However, this known approach does not take into consideration physiology during the actual recovery process, which happens from the end of the exercise training session or race and includes sleep as central moment. According to the present invention, the physiology of recovery is accounted for and in particular sleep. A model of recovery may be made based on the exercise episodes, which model may be compared to the actual responses during sleep (and, optionally, rest). By doing so the training-recovery status is kept on check and any unbalance is detected. This information may be promptly communicated, e.g. to the user (such as an athlete and/or coach).

Known solutions monitoring recovery from exercise do further not account for sleep architecture changes caused by the exercise itself. According to embodiments of the present invention an algorithm may be used that profiles the user, classifies the user's training and forecasts the effects of the interaction user-training regime on the sleep architecture. This model then compares the forecasted effects on sleep to the actual sleep architecture and from this comparison determines the presence of complete or incomplete recovery.

In this context, sleep architecture refers to the sleep stages and the cycle infrastructure of sleep. Sleep architecture is not only about whether a user is in REM (rapid eye movement) or non-REM sleep, but it is also about how these stages follow each other constituting a structural pattern. For instance, knowing that a user had e.g. one hour of REM sleep and two hours of non-REM deep sleep (which would not be sleep architecture) is something else than knowing when these stages occurred and in which sequence these stages occurred.

The sensor for measuring/acquiring one or more physiological signals and/or actual exercise data of a subject may include one or more of heart rate sensor, a respiration sensor, an SpO2 sensor, a motion sensor, an accelerometer, a force sensor, a pressure sensor, an electromyography sensor, a temperature sensor, a calorie expenditure sensor, a global positioning system sensor, a remote camera, a radar, a speed measurement sensor, a torque measurement sensor, an ECG sensor, an EEG sensor, a muscle tone sensor, a sweat level sensor, a stress level sensor, a skin conductance sensor, and a galvanic skin response sensor.

The subject data source for inputting and/or storing subject data including information on one or more characteristics of the subject may include one or more of a user input device, a database and the user interface, and it may be configured to input and/or store, as subject data, one or more of age, gender, body dimensions, weight, ethnicity, activity level, medication, diet, stress level, pre-existing conditions, and injuries.

The processor may further be configured to estimate second recovery information on the subject's recovery from the one or more physiological signals and/or the actual exercise data measured during the exercise state and the obtained subject-related information, and determine final recovery information on the subject's recovery from the first recovery information and the second recovery information. This further improves the accuracy of the determined recovery information.

In another embodiment the processor may further be configured to further distinguish another state — a rest state — of the subject, in particular to determine from the obtained one or more physiological signals and/or the obtained actual exercise data if the subject is a rest state in which the subject is resting, estimate, from the one or more physiological signals and/or actual exercise data measured during a rest state and the obtained subject-related information, reference recovery information on the subject's recovery, and determine final recovery information on the subject's recovery from the first recovery information and the reference recovery information. This further improves the accuracy of the determined recovery information as well.

In an embodiment the processor is configured to determine the first recovery information by comparing the actual sleep architecture and the estimated sleep architecture, estimating recovery gap information indicating a recovery gap of the subject by comparing the result of the comparison with a reference sleep architecture, in particular a normative sleep architecture stored in a database or a sleep architecture generated from the subject-related information and from physiological signals and/or actual exercise data of the subject while the subject was resting or sleeping, and updating the final recovery information based on the estimated recovery gap information. A recovery gap can be defined as a residual level of fatigue, directly associated with the exercise training event, of any physiological function, such as cardio-pulmonary, muscular, mental (cognitive), etc.

The processor may further be configured to determine the actual sleep architecture and/or to estimate the estimated sleep architecture by determining or estimating sleep stage information on one or more types of sleep stages of the subject during a sleep state and time information on the sleep stages, in particular information on duration and/or sequence of sleep stages.

Preferably, the processor is configured to estimate the estimated sleep architecture by use of a model, in particular a bio-statistical model.

In an implementation the processor may be configured to determine if the subject is in an exercise state or in a sleep state (or, optionally, in a rest state) by comparing one or more of the obtained physiological signals and/or actual exercise data against respective thresholds or ranges, in particular thresholds or ranges selected according to the subject-related information.

The recovery information may include one or more pieces of information that may be useful for a user, such as an athlete or coach. In a corresponding embodiment the processor may be configured to determine first recovery information by determining one or more of how long the subject should wait to perform a next exercise, which exercise the subject may perform as next exercise or should not perform as next exercise, and with which intensity and/or duration the subject may perform a next exercise.

Further, in an embodiment the processor may be configured to determine the first recovery information by estimating virtual exercise data of the subject from the actual sleep architecture, said virtual exercise data indicating how the exercise of the subject looks like based on the actual sleep architecture, and determining a correction value for correcting the determined subject's recovery by comparing the estimated virtual exercise data with the obtained actual exercise data of the subject. For instance, the processor may be configured to correct the determined subject's recover by the determined correction value by use of a Kalman filter or a maximum likelihood approach. This may further improve the determination and monitoring of recovery.

BRIEF DESCRIPTION OF THE DRAWINGS

These and other aspects of the invention will be apparent from and elucidated with reference to the embodiment(s) described hereinafter. In the following drawings

    Fig. 1 shows a schematic diagram of an embodiment of a system according to the present invention.
    Fig. 2 shows a schematic diagram of an embodiment of a device according to the present invention.
    Fig. 3 shows a schematic diagram of an embodiment of a method according to the present invention.
    Fig. 4 shows a schematic diagram of another embodiment of a device according to the present invention.

DETAILED DESCRIPTION OF THE EMBODIMENTS

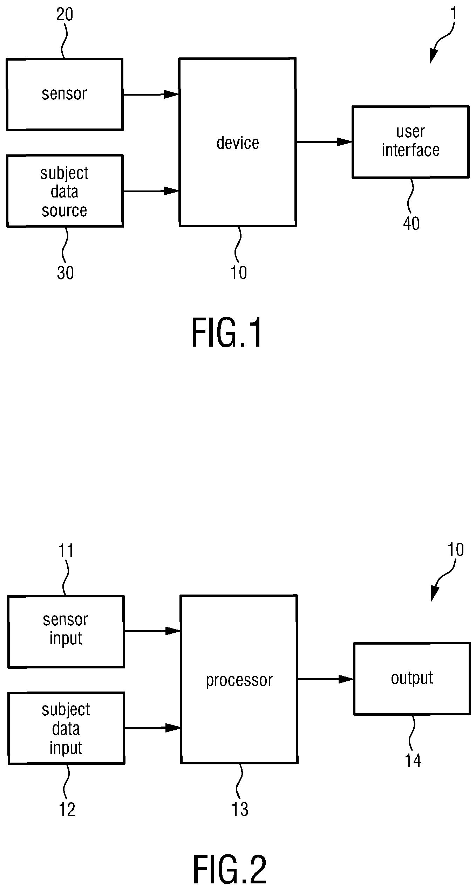

Fig. 1 shows a schematic diagram of an embodiment of a system 1 for generating information on recovery of a subject (e.g. a patient, an athlete, etc.) according to the present invention.

The system 1 comprises a sensor 20 configured to measure one or more physiological signals and/or actual exercise data of the subject. Different kinds of sensor may be used for this purpose, such as a heart rate sensor, a respiration sensor, an SpO2 sensor, a motion sensor, an accelerometer, a force sensor, a pressure sensor, an electromyography sensor, a temperature sensor, a calorie expenditure sensor, a global positioning system sensor, a remote camera, a radar, a speed measurement sensor, a torque measurement sensor, an ECG sensor, an EEG sensor, a muscle tone sensor, a sweat level sensor, a stress level sensor, a skin conductance sensor, and a galvanic skin response sensor. Physiological signals may thus e.g. be heart rate (HR), heart rate variability (HRV), HRV features, inter-beats intervals, respiration information (such as respiration rate (RR), inhalation volume, exhalation volume, inhalation duration, exhalation duration, etc.), muscle tone, skin conductivity, sweat response, SpO2, temperature, calorie expenditure, etc. Actual exercise data may be any data that indicate the current subject's exercise/activity, such as movement data (movement speed, type of movement, duration of movement), information on position/posture, position/posture changes, type of exercise, duration of exercise, intensity of exercise, etc. Generally, any information/signal that allows tracking the subject's training workload and sleep architecture may be measured.

The system 1 further comprises a subject data source 30 configured to input and/or store subject data including information on one or more characteristics of the subject. The subject data source may be a user input device (e.g. touchscreen, computer, tablet, smartphone, keypad, etc.), a database, etc. The subject data may include, as information on one or more characteristics of the subject, one or more of age, gender, body dimensions, weight, ethnicity, activity level (e.g. sport, exercise), medication, diet, stress level, pre-existing conditions, injuries, etc.

The system 1 further comprises a device 10 configured to generate information on recovery of a subject using the measured one or more physiological signals and the subject data. Details of the device 10 will be described below. The device 10 may e.g. be implemented as or in a processor or computer, in software and/or hardware. The information on recovery may include one or more different pieces of information, such as information on how long the subject should wait to perform a next exercise, which exercise the subject may perform as next exercise or should not perform as next exercise, and with which intensity and/or duration the subject may perform a next exercise. Further, the system 1 may be able to predict how a certain planned exercise training would impact of the recovery, i.e., to predict what is the impact on recovery and performance of a given forecasted exercise.

The system 1 further comprises a user interface 40 configured to issue the generated information on recovery.

The user interface 40 may generally be any means that outputs the generated information on recovery in visual or audible form, e.g. in text form, as image or diagram, as sound or spoken words, etc. For instance, the user interface 40 may be a display, a loudspeaker, a touchscreen, a computer monitor, the screen of a smartphone or tablet, etc.

Fig. 2 shows a schematic diagram of an embodiment of a device 10 for generating information on recovery of a subject according to the present invention.

The device 10 comprises a sensor input 11 configured to obtain one or more physiological signals and/or actual exercise data of the subject. The sensor input 11 may be directly coupled or connected to the sensor 20 or may obtain (i.e. retrieve or receive) these signals/data from a storage, buffer, network, or bus, etc. The sensor input 11 may thus e.g. be (wired or wireless) communication interfaces or data interfaces, such as a Bluetooth interface, WiFi interface, LAN interface, HDMI interface, direct cable connect, or any other suitable interface allowing signal transfer to the device.

The device 10 further comprises a subject data input 12 configured to obtain subject-related information on one or more characteristics of the subject. The subject data input 12 may be directly coupled or connected to the subject data source 30 or may obtain (i.e. retrieve or receive) these signals/data from a storage, buffer, network, or bus, etc. The subject data input 12 may thus e.g. be (wired or wireless) communication interfaces or data interfaces, such as a Bluetooth interface, WiFi interface, LAN interface, HDMI interface, direct cable connect, or any other suitable interface allowing signal transfer to the device.

The device 10 further comprises a processor (or processing unit) 13 configured to generate information on recovery of the subject using the obtained one or more physiological signals and the obtained subject data. The processor may be any kind of means configured to process the obtained signals/data and determine the information on recovery there from. It may be implemented in software and/or hardware, e.g. as a programmed processor or computer or app on a user device such as a smartphone, smartwatch, tablet, laptop, PC, workstation, etc.

The device 10 further comprises an output 14 configured to output the generated information on recovery. The output 14 may generally be any interface that provides the generated information on recovery, e.g. transmits it to another device or provides it for retrieval by another device (e.g. a smartphone, computer, tablet, etc.). It may thus generally be any (wired or wireless) communication or data interface.

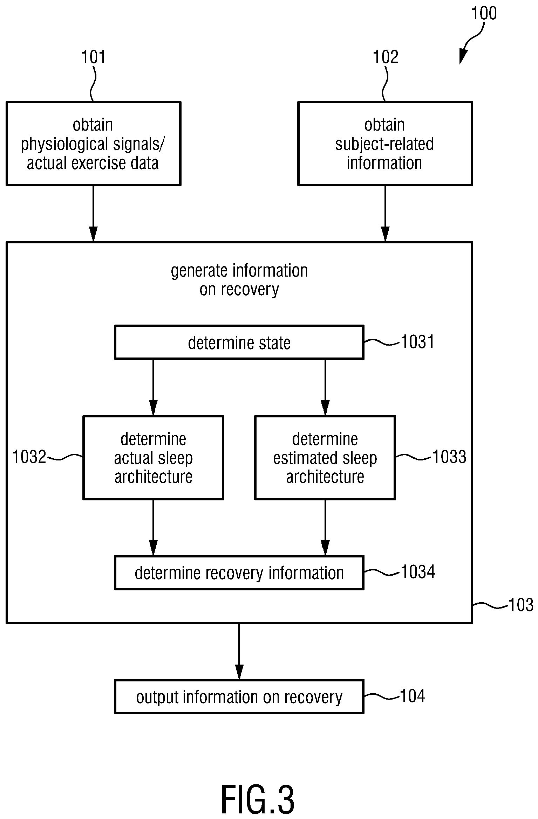

Fig. 3 shows a schematic diagram of a first embodiment of a method 100 for determining generating information on recovery of a subject according to the present invention. The steps of the method may be carried out by the device 10, wherein the main steps of the method are carried out by the processor 13. The method may e.g. be implemented as computer program running on a computer or processor.

In a first step 101 oone or more physiological signals and/or actual exercise data of the subject are obtained. In a second step 102 subject-related information on one or more characteristics of the subject are obtained. In a third step 103 information on recovery of the subject is generated using the obtained one or more physiological signals and/or one or more actual exercise data and the obtained subject data. In a fourth step 104 the generated information on recovery is outputted.

The step 103 of generating the information on recovery includes a first sub-step 1031 of determining from the obtained one or more physiological signals and/or the obtained actual exercise data if the subject is in an exercise state in which the subject performs an exercise or in a sleep state in which the subject is sleeping. In a second sub-step 1032 it is determined, from the one or more physiological signals and/or the one or more actual exercise data measured during a sleep state and the obtained subject-related information, an actual sleep architecture of the subject. At the same time or before or after, from the one or more physiological signals and/or the one or more actual exercise data measured during an exercise state and the obtained subject-related information, an estimated sleep architecture of the subject is estimated in a third sub-step 1033. Finally, in sub-step 1034 first recovery information on the subject's recovery is determined based on a comparison of the actual sleep architecture and the estimated sleep architecture.

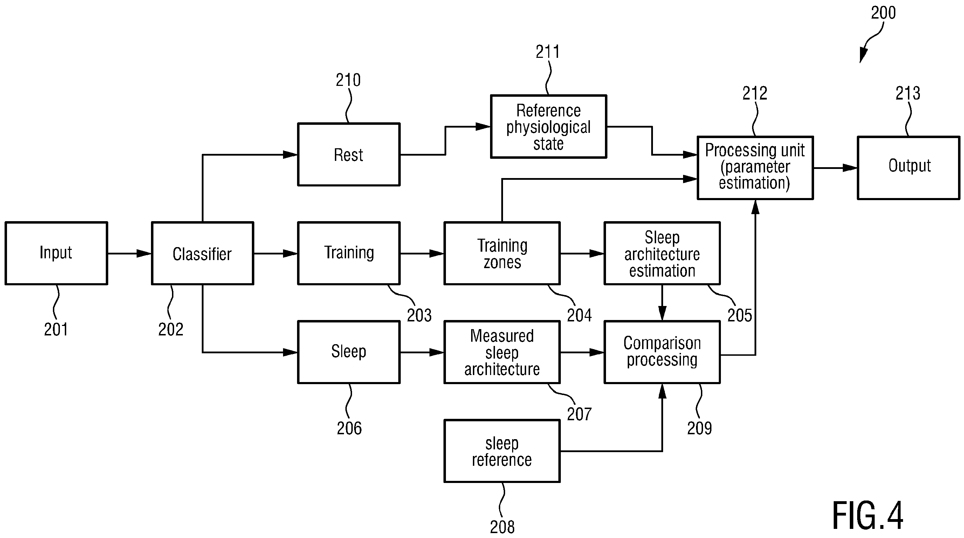

There are multiple embodiments of the claimed device, system and method available, according to which one or more additional elements or processing steps are provided that may or may not be used in certain combinations. Fig. 4 shows a schematic diagram of a particular embodiment of a device 200 according to the present invention. The elements of the device 200 may be used in this combination, but one or more elements may be omitted in other embodiments and/or may be used in other combinations in other embodiments of the disclosed device.

At the input 201 the physiological signals, actual exercise data and subject-related information are provided, e.g. received or retrieved from a memory or database or directly from respective means like sensors and input interface.

A classifier 202 classifies, e.g. based on a threshold or waveform pattern, the activity in rest (or non-exercise), exercise, or sleep. A simple implementation of the classifier 202 makes use of one or more thresholds. For example, using HR as physiological signal, training is classified based on a personalized value of HR (e.g. $120 \pm 2$ bpm) and for sleep HR is below a personalized value (e.g. $55 \pm 5$ bpm) for at least 2 min. More accurate ways for classification will be used in more elaborate implementations. For instance, a more accurate threshold-based classifier may use two parameters and corresponding thresholds, such as HR and an accelerometer signal (ACC) indicating subject motion.

If it is determined that the subject is in exercise (or training) state (block 203), exercise data (actual activity data) is mainly used to determine characteristics such as, workload, duration, type (volume, intensity). Such exercise data may be used in two ways: i) to estimate recovery; and ii) to estimate the sleep architecture.

In this context, estimating recovery substantially means to determine how long the user should wait to be able to perform another exercise event (being this a training or a competition) in order to avoid over-fatiguing (defined as the additive effect of a fatiguing episode, such as a training event or a competition, on top of a pre-existent fatiguing episode which did not yet cease).

Further, in this context, sleep architecture is a combination of sleep phases (or stages) information, their duration and their succession. Each person has a pattern, but those patterns are subjected to disruption depending on what the person has been doing when awake and when the person goes to sleep and for how long. Training is therefore a major influencing factor of sleep. A bio-statistical model (e.g. a mixed linear model) may be used to estimate sleep architecture based on the exercise data (block 205).

In block 204 training zones are derived for instance by knowing the age, sex and fitness level (CrF) of the user. The fitness level can be derived from the usage data, or in a number of known ways (e.g. protocol based running, or cycling fitness test). Usage data fitness assessment, as e.g. described in US 10470704 B2, is the preferred option as it requires the user only to wear the device for a minimum of one exercise session. When age, sex and fitness level are known any given exercise can be sub-divided into personalized training zones. For example, a 30 minutes running session, for simplicity at a constant speed at 10 km/h, with only two ascending and descending phases (0 km/h to 10 km/h and 10 km/h to 0 km/h), both for simplicity of 60 seconds each, will result in a total of 30 seconds spent in zone 1, 30 seconds spent in zone 2 and 28 minutes and 30 seconds spent in zone 3. Each zone has a different contribution to fatiguing the system, for instance zone 1 has a contributing coefficient of 0.2; zone 2 has a contributing coefficient of 0.4; zone 3 has a contributing coefficient of 0.6; zone 4 has a contributing coefficient of 0.8; zone 5 has a contributing coefficient of 1. Those coefficients differ per physiological system (e.g. cardio-respiratory, muscular, metabolic ...). The body needs to recover from fatigue of these physiological systems determine a unique pattern of how exercise training influences sleep and its architecture. This unique pattern is derived to estimate the sleep architecture (block 205).

If it is determined that the subject is in sleep state (block 206), the input signal/data classified as sleep data is used to assess the real (or actual/measured) sleep architecture (block 207). Optionally, a sleep look-up reference (208), e.g. taken from a database or literature, can be used, e.g. to obtain reference sleep values, to determine a possible discrepancy between the real (or actual/measured) sleep architecture with the sleep reference (block 209). Based thereon a first recovery information on the subject's recovery may be determined (and outputted). The database optionally used to obtain a sleep look-up reference can be generated within the sleep data of the user, e.g. by aggregating all the nights labelled by an algorithm and/or by the user as resting night. When this database (user based) is large enough, outliers can be discarded, so that the sleep reference becomes more and more reliable with usage.

Optionally, second recovery information on the subject's recovery may be determined in block 204 from the one or more physiological signals and/or the actual exercise data measured during the exercise state and the obtained subject-related information. The first recovery information (output of block 209) and the second recovery information (output of block 204) may then be used to determine final recovery information on the subject's recovery (block 212), e.g. by averaging the first and second recovery information.

If it is (optionally) determined that the subject is in non-exercise (rest) state, the input data classified as non-exercise is used as reference recovery information (reference physiological state) (block 211). Final recovery information may then be determined (block 212) from the first recovery information (output of block 209) and the reference recovery information (output of block 211) (and, optionally the second recovery information), e.g. by averaging the different pieces of recovery information.

Sleep architecture can be measured (block 207) unobtrusively with the same sensor setup used for measuring training, or with alternative sensors that are known to provide this information. For this purpose, known algorithms based e.g. on heart rate variability and/or respiratory variability can be used, which can be measured comfortably and unobtrusively on relevant nights, for example using reflective photoplethysmography (e.g. worn on the wrist), ballistocardiography (e.g. with a sensor mounted on the bed, on top of, or under the mattress), or even RF-based (e.g. with a Doppler radar on the night-table). All of these sensors can provide physiological information about body movements, cardiac activity and/or respiratory activity.

In turn, pre-trained machine learning algorithms can be used to detect sleep stages either with end-to-end deep neural network models, directly from the raw signal, or with a first step of manually engineered feature extraction (e.g. heart rate variability, respiratory variability) after which a classifier (e.g. using recurrent neural networks) can be used to translate these features in to the most likely sleep stages for each time point in the night.

To determine a possible discrepancy between the real (or actual/measured) sleep architecture with the sleep reference in block 209 a comparison may be used. In this step the exercise-derived sleep architecture estimation is compared with the measured sleep architecture and both can be compared with reference normative data. Nowadays, sleep is monitored not only in sleep clinics but also more unobtrusively. Thus, there are databases available where these normative data are derived or derivable. The same product could be used to build such database if not yet present. In any case, the simplest way to build this comparison norm is the data of the users themselves when they are not training.

The comparison norm may be updated anytime new "resting" data is collected. Labeling of the resting data can be achieved by using the classifier. Normative data of two consecutive resting day will replace normative data from one resting day, normative data from three consecutive resting days will replace two or less. This may be done if a large pre-existent dataset is absent.

This comparison step may thus be used to identify a possible sleep derived recovery gap. The possible recovery gap is then used to update the recovery determination done from the training and the resting data.

In an exemplary implementation it is supposed that a vector T(1×N) contains all training features including the heart rate zones. Similarly, a vector S contains the measured sleep architecture during the night. Using S, an estimate of the training features $\hat{T}$ may be derived (e.g. using Mixed Effects Modeling):

$$S = \begin{bmatrix} S_1 \\ S_2 \\ \vdots \\ S_m \end{bmatrix} \longrightarrow \hat{T} = \begin{bmatrix} T_1 \\ T_2 \\ \vdots \\ T_n \end{bmatrix}$$

Comparison of the body's experience of the workload $\hat{T}$ with the actual performed workload T hints towards the body's recovery status R, with R being equal to:

$$R = TRIMP - T\hat{R}IMP$$

*TRIMP* is a single integer value reflecting the overall intensity of the training, derived from the training feature vector T. Therefore, R > 0 corresponds to a higher perceived than actual workload, reflecting little recovery. Contrarily, values of R < 0 correspond to sufficient recovery from training. This information may then he used to predict a new recovery status.

In the embodiment described above, T (a vector which characterizes a training (or competition) event) is derived from the actual training data, as T1 may be the training duration, T2 may be the training intensity (e.g. HR max), etc. In this embodiment a $\hat{T}$ vector may be modelled from how the user slept. That means that $\hat{T}$ has the same type (number) of characteristics as the T vector (e.g. $\hat{T}$1 = training duration, $\hat{T}$2= training intensity (e.g. HR max), etc. $\hat{T}$n...) but it is derived only after a night of sleep.

An example shall be given to explain this: For instance, if the user trains today at 16:00 until 16:30, then the T vector is available as soon as the session is finished (e.g.16:30). T = [30min; 150 bpm; ....; 7.5 km] for instance. There are different methods known how these training characteristics can be used to estimate the recovery (e.g. using a Banister model). However, this is based on the assumption that nothing strange happens from the end of the training until those models predict that fatigue = 0. This is not happening in reality so that typically these models are inaccurate.

If the user goes to sleep at night, on the next morning a $\hat{T}$ matrix is computed according to the present invention based on how the user has slept (sleep architecture vector S). For the sake of argument that the user did not sleep well, or at least the restorative part of his/her night was sub-optimal, $\hat{T}$ = [60min; 155 bpm; ....; 13 km]. From sleep alone it looks as if the user had been running 1 h instead of 30 min, the user's HR is elevated and less efficient, as the user covered fewer km. The vector R can now be computed as, for example, the mere difference between T and $\hat{T}$.

S in the simplest way is as described above. It is a matrix where the columns are the number of sleep architecture features (m) and the rows are the number corresponding to the length of T and $\hat{T}$(n), basically to form a system of regressions.

TRIMP is a specific solution. Basically, TRIMP is the product of two elements of the vector T, precisely Exercise Duration * Exercise Intensity. In the example above, duration, intensity, and distance are used. The same example without distance corresponds to TRIMP.

The result of this comparison step is a new recovery estimation that accounts not only the exercise but also the influence of sleep and the need of sleep based on a given activity. Via e.g. a maximum likelihood approach, the parameters for modelling the overall recovery using a state-space model are estimated (block 212). A Kalman filter may e.g. be used to correct the estimation of recovery based on S as input, T as measure, $\hat{T}$ as estimated measure and R as state, over time. Hereby, it holds that recovery = A $R_{(t-1)}$ + B $S_{(t)}$+ $K_{(t)}$ ($T_{(t)}$ — C (A $R_{(t-1)}$ + B $T_{(t)}$)), where A matrix is the state matrix, B matrix is input matrix , C matrix is the output matrix, and K is the Kalman filter.

The parameter values become individual weights, thereby enabling for personalized modelling of the recovery status. The information about the overall recovery may then be output e.g. for coaching a user (block 213).

In an embodiment a parameter estimation of a Banister systems model describing recovery may be used to estimate recovery. The Banister model is defined by the sum of two components fitness (CrF) and fatigue (Fa). The Banister model estimates performance (P) as

$$P(t) = P(0) + CrF - Fa$$

where fitness is:

$$CrF = k_1 \sum_{s=0}^{t-1} e^{-(t-s)/t_1} w(s)$$

and where fatigue is:

$$Fa = k_2 \sum_{s=0}^{t-1} e^{-(t-s)/t_2} w(s)$$

Performance is known as well as CrF as described above $k_1$ and $k_2$ are time constants and $w(s)$ is a given workload referred above asTRIMP:

$$Fa = P(0) - P(t) + CrF$$

Recovery in this embodiment is:

$$F = -F_a + 100$$

The recovery reference is: $[F_1, F_2, ..., F_n]$
The estimated recovery is: $[\hat{F}_1, \hat{F}_2, ..., \hat{F}_n]$
The parameter estimation may result in:

$$[F_1, F_2, ..., F_n]$$

$$[\hat{F}_1, \hat{F}_2, ..., \hat{F}_n]$$

$$\frac{df}{dt} = -\left(\frac{1}{p(\theta)}\right) f + u$$

$$p(\theta) = \sum_{i=1}^{N} \theta_i (F_i - \hat{F}_i)$$

Set of individual 'weights'
$[\theta_1, \theta_2, ..., \theta_n]$

where p(θ) is a given parameter in function of an individual weight.
Thus, according to embodiments of the present invention the physiology of recovery is accounted for and in particular sleep. A model of recovery is made based on the exercise episodes and it is compared to the actual responses during sleep (and optionally rest). The training-recovery status can thus be monitored and checked and any unbalance can be detected.
While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.
In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (10) for generating information on recovery of a subject, said device comprising

   a sensor input (11) configured to obtain one or more physiological signals and/or actual exercise data of a subject;
   a subject data input (12) configured to obtain subject-related information on one or more characteristics of the subject;
   a processor (13) configured to generate information on recovery of the subject using the obtained one or more physiological signals and the obtained subject data; and
   an output (14) configured to output the generated information on recovery,
   wherein the processor (13) is configured to

   - determine from the obtained one or more physiological signals and/or the obtained actual exercise data if the subject is in an exercise state in which the subject performs an exercise or in a sleep state in which the subject is sleeping,
   - determine, from the one or more physiological signals and/or the one or more actual exercise data measured during a sleep state and the obtained subject-related information, an actual sleep architecture of the subject,
   - estimate, from the one or more physiological signals and/or the one or more actual exercise data measured during an exercise state and the obtained subject-related information, an estimated sleep architecture of the subject, and
   - determine first recovery information on the subject's recovery based on a comparison of the actual sleep architecture and the estimated sleep architecture.

2. Device as claimed in any one of the preceding claims,
   wherein the processor (13) is further configured to

   - estimate second recovery information on the subject's recovery from the one or more physiological signals and/or the actual exercise data measured during the exercise state and the obtained subject-related information, and
   - determine final recovery information on the subject's recovery from the first recovery information and the second recovery information.

3. Device as claimed in any one of the preceding claims,
   wherein the processor (13) is further configured to

   - determine from the obtained one or more physiological signals and/or the obtained actual exercise data if the subject is a rest state in which the subject is resting,
   - estimate, from the one or more physiological signals and/or actual exercise data measured during a rest state and the obtained subject-related information, reference recovery information on the subject's recovery, and
   - determine final recovery information on the subject's recovery from the first recovery information and the reference recovery information.

4. Device as claimed in claim 2 or 3,
   wherein the processor (13) is configured to determine the first recovery information by

   - comparing the actual sleep architecture and the estimated sleep architecture,
   - estimating recovery gap information indicating a recovery gap of the subject by comparing the result of the comparison with a reference sleep architecture, in particular a normative sleep architecture stored in a database or a sleep architecture generated from the subject-related information and from physiological signals and/or actual exercise data of the subject while the subject was resting or sleeping, and
   - updating the final recovery information based on the estimated recovery gap information.

**5.** Device as claimed in any one of the preceding claims,
wherein the processor (13) is configured to determine the actual sleep architecture and/or to estimate the estimated sleep architecture by determining or estimating sleep stage information on one or more types of sleep stages of the subject during a sleep state and time information on the sleep stages, in particular information on duration and/or sequence of sleep stages.

**6.** Device as claimed in any one of the preceding claims,
wherein the processor (13) is configured to estimate the estimated sleep architecture by use of a model, in particular a bio-statistical model.

**7.** Device as claimed in any one of the preceding claims,
wherein the processor (13) is configured to determine if the subject is in an exercise state or in a sleep state by comparing one or more of the obtained physiological signals and/or actual exercise data against respective thresholds or ranges, in particular thresholds or ranges selected according to the subject-related information.

**8.** Device as claimed in any one of the preceding claims,
wherein the processor (13) is configured to determine first recovery information by determining one or more of how long the subject should wait to perform a next exercise, which exercise the subject may perform as next exercise or should not perform as next exercise, and with which intensity and/or duration the subject may perform a next exercise.

**9.** Device as claimed in any one of the preceding claims,
wherein the processor (13) is configured to determine the first recovery information by

- estimating virtual exercise data of the subject from the actual sleep architecture, said virtual exercise data indicating how the exercise of the subject looks like based on the actual sleep architecture, and
- determining a correction value for correcting the determined subject's recovery by comparing the estimated virtual exercise data with the obtained actual exercise data of the subject.

**10.** Device as claimed in claim 9,
wherein the processor (13) is configured to correct the determined subject's recover by the determined correction value by use of a Kalman filter or a maximum likelihood approach.

**11.** System for generating information on recovery of a subject, said system comprising

a sensor (20) configured to measure one or more physiological signals and/or actual exercise data of a subject;
a subject data source (30) configured to input and/or store subject data including information on one or more characteristics of the subject;
a device (10) as claimed in any one of the preceding claims configured to generate information on recovery of a subject using the measured one or more physiological signals and the subject data; and
a user interface (40) configured to issue the generated information on recovery.

**12.** System as claimed in claim 11,
wherein the sensor (20) includes one or more of heart rate sensor, a respiration sensor, an SpO2 sensor, a motion sensor, an accelerometer, a force sensor, a pressure sensor, an electromyography sensor, a temperature sensor, a calorie expenditure sensor, a global positioning system sensor, a remote camera, a radar, a speed measurement sensor, a torque measurement sensor, an ECG sensor, an EEG sensor, a muscle tone sensor, a sweat level sensor, a stress level sensor, a skin conductance sensor, and a galvanic skin response sensor.

**13.** System as claimed in claim 11 or 12,

wherein the subject data source (30) includes one or more of a user input device, a database and the user interface (40), and
wherein the subject data source (30) is configured to input and/or store one or more of age, gender, body dimensions, weight, ethnicity, activity level, medication, diet, stress level, pre-existing conditions, and injuries.

**14.** Method for generating information on recovery of a subject, said device comprising

obtaining one or more physiological signals and/or actual exercise data of a subject;

obtaining subject-related information on one or more characteristics of the subject;

generating information on recovery of the subject using the obtained one or more physiological signals and/or one or more actual exercise data and the obtained subject data; and

outputting the generated information on recovery,

wherein generating the information on recovery includes

- determining from the obtained one or more physiological signals and/or the obtained actual exercise data if the subject is in an exercise state in which the subject performs an exercise or in a sleep state in which the subject is sleeping,
- determining, from the one or more physiological signals and/or the one or more actual exercise data measured during a sleep state and the obtained subject-related information, an actual sleep architecture of the subject,
- estimating, from the one or more physiological signals and/or the one or more actual exercise data measured during an exercise state and the obtained subject-related information, an estimated sleep architecture of the subject, and
- determining first recovery information on the subject's recovery based on a comparison of the actual sleep architecture and the estimated sleep architecture.

15. Computer program comprising program code means for causing a computer to carry out the steps of the method as claimed in claim 14 when said computer program is carried out on the computer.

20

sensor

subject
data
source

30

device

10

1

user
interface

40

FIG.1

11

sensor
input

subject
data
input

12

processor

13

10

output

14

FIG.2

100

101

obtain
physiological signals/
actual exercise data

102

obtain
subject-related
information

generate information
on recovery

| determine state |

1031

| determine
actual sleep
architecture | | determine
estimated sleep
architecture |

1032

1033

| determine recovery information |

1034

103

| output information on recovery |

104

FIG.3

FIG.4

EP 4 170 666 A1

## EUROPEAN SEARCH REPORT

Application Number

EP 21 20 4279

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/374569 A1 (BRESLOW EMILY RACHEL [US] ET AL) 29 December 2016 (2016-12-29) * paragraphs 3-7, 140, 159, 161, 187, 248-259, 71; figure 15A * | 1-15 | INV. G16H20/30 G16H50/20 G16H50/30 |
| A | MORTON R. H. ET AL: "Modeling human performance in running", JOURNAL OF APPLIED PHYSIOLOGY, vol. 69, no. 3, 30 September 1990 (1990-09-30), pages 1171-1177, XP055908478, US ISSN: 8750-7587, DOI: 10.1152/jappl.1990.69.3.1171 | 1-15 | |

TECHNICAL FIELDS SEARCHED (IPC)

G16H

The present search report has been drawn up for all claims

| Place of search | Date of completion of the search | Examiner |
|---|---|---|
| Munich | 5 April 2022 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 4279

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2022

| Patent document cited in search report | Publication date | Patent family member(s) | Publication date |
|---|---|---|---|
| US 2016374569 A1 | 29-12-2016 | CA 2883852 A1 | 13-03-2014 |
| | | EP 2892421 A1 | 15-07-2015 |
| | | US 2014073486 A1 | 13-03-2014 |
| | | US 2014309542 A1 | 16-10-2014 |
| | | US 2014323827 A1 | 30-10-2014 |
| | | US 2014323828 A1 | 30-10-2014 |
| | | US 2014323880 A1 | 30-10-2014 |
| | | US 2014343372 A1 | 20-11-2014 |
| | | US 2014350356 A1 | 27-11-2014 |
| | | US 2016129310 A1 | 12-05-2016 |
| | | US 2016360985 A1 | 15-12-2016 |
| | | US 2016367187 A1 | 22-12-2016 |
| | | US 2016374569 A1 | 29-12-2016 |
| | | US 2017188847 A1 | 06-07-2017 |
| | | US 2021210188 A1 | 08-07-2021 |
| | | WO 2014039567 A1 | 13-03-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10470704 B2